# EUROPEAN PATENT APPLICATION

(11) **EP 4 458 842 A1**
(43) Date of publication of application: **06.11.2024**
(21) Application number: 22901639.9
(22) Date of filing: 18.11.2022
(51) Int. Cl.: C07K 14/005, A61K 48/00, A61P 9/12, C12N 15/86, A61P 11/00

(54) **MUTANT OF ADENO-ASSOCIATED VIRUS CAPSID PROTEIN**

(30) Priority: 30.11.2021 KR 20210168583; 23.09.2022 KR 20220120442
(71) Applicant: Glugenetherapeutics Inc., Daejeon 34028 (KR)
(72) Inventor: JANG, Jae-Hyung, Seoul 07997 (KR); KIM, Yoo Jin, Seoul 06625 (KR)
(74) Representative: Schiweck Weinzierl Koch Patentanwälte Partnerschaft mbB
(86) International application number: PCT/KR2022/018287
(87) International publication number: WO 2023/101281

(57) **Abstract**

The present invention relates to a mutant of the adeno-associated virus serotype 1 (AAV1) capsid protein, a nucleic acid coding for the mutant of the AAV1 capsid protein, a recombinant AAV1 vector carrying the nucleic acid coding for the mutant of the AAV1 capsid protein, and a pharmaceutical composition comprising the vector, wherein the mutant has cysteine substituted for serine at position 430 and valine substituted for isoleucine at position 647 in comparison with the amino acid sequence of the wild-type AAV capsid protein. Specifically, when delivered in an aerosol state through the trachea, the recombinant virus vector carrying a nucleic acid coding for the mutant of the AAV1 capsid protein enhances transgene expression in pulmonary vessel targeting cells and thus is useful for preventing or treating pulmonary arterial hypertension.

## Description

### [Technical Field]

The present invention relates to a mutant of an adeno-associated virus (AAV) capsid protein, and particularly, to a recombinant virus vector including a mutant of an AAV1 capsid protein is useful for the expression of a transgene in target pulmonary vessel cells when intratracheally delivered in an aerosol state.

### [Background Art]

To make gene therapy effective, the development of gene transfer technology that can deliver therapeutic genes to desired target cells and achieve high expression efficiency is a top priority.

Among these gene delivery technologies, AAVs are non-pathogenic viruses that have no side effects on infiltrated infected cells and a low probability of causing mutations in the genetic information of target cells, making it superior in terms of safety compared to other gene therapy technologies.

Adeno-associated viruses (AAV) are non-enveloped single-stranded DNA viruses that can infect both dividing and non-dividing cells. AAVs can replicate only in the presence of a helper virus and are non-pathogenic to humans. With these characteristics, an AAV is a useful method of introducing genes into various cells and is used as a useful vector for gene therapy.

It is known that AAVs have various serotypes and that the characteristics of a host or virus differ depending on the serotype. Serotype 2 (AAV2) is a serotype that has been widely studied for a long time and can infect various cells. Serotype 1 (AAV1), Serotype 5 (AAV5), and Serotype 6 (AAV6) are serotypes with more tissue infection specificity. It is known that AAV1 has high gene introduction efficiency with respect to muscles, the liver, the trachea, and the central nervous system, AAV5 high gene introduction efficiency with respect to the central nervous system, the liver, and the retina, and AAV6 high gene introduction efficiency with respect to the heart, muscles, and the liver. Although the characteristics of gene delivery to specific tissue vary depending on the serotype, it is still easy to transfer to other tissues, so it may be necessary to develop a new AAV vector that can improve stability and efficiency by improving tissue specificity.

Meanwhile, pulmonary arterial hypertension is a disease in which the pulmonary arterioles narrow for no apparent reason, leading to an increase in pulmonary arterial pressure and a subsequent decrease in right ventricular function. The main symptoms of pulmonary arterial hypertension, such as shortness of breath, and dizziness, are common in everyday life, so it is easy to overlook them or think them as symptoms of another disease. For this reason, there is often a delay before a diagnosis of pulmonary arterial hypertension is made for a patient.

In fact, according to a survey conducted by the Korean Centers for Disease Control and Prevention, the time for a patient to be accurately diagnosed with pulmonary arterial hypertension was 1.5 years on average. In addition, for accurate diagnosis, an expensive cardiac catheterization test of inserting a wire into the body was required.

Blood is not transmitted smoothly from the heart to the lungs, which leads to breathing difficulties and heart failure, and in severe cases, it can result in death. Despite steady advancements in medical technology, the 5-year survival rate for patients with pulmonary arterial hypertension is only about 50%. Due to this very poor prognosis, early diagnosis and treatment are important.

Although attempts have been made to improve gene introduction efficiency by modifying AAV capsid proteins, no study on adeno-associated viruses targeting pulmonary vessels has yet been reported.

### [Related Art Documents]

### [Patent Documents]

(Patent Document 1) International Publication No. WO/2017/201121 (published November 23, 2017)
(Patent Document 2) Japanese Laid-open Publication No. 2021-0010372 (published February 04, 2021)

### [Disclosure]

### [Technical Problem]

Therefore, as a result of intensively trying to solve the above problem, the inventors developed a protein variant including a novel capsid of adeno-associated virus (AAV) serotype 1, as a basic backbone, which targets pulmonary vessels and has the potential to deliver a gene therapeutic agent that addresses the underlying genetic cause of pulmonary arterial hypertension. Thus, the present invention was completed.

### [Technical Solution]

Therefore, the present invention is directed to providing a mutant of an AAV1 capsid protein for improving the efficiency of gene introduction into target cells and/or genetic information expression efficiency by a recombinant AAV.

The present invention is also directed to providing a nucleic acid that encodes the mutant of an AAV1 capsid protein.

The present invention is also directed to providing a recombinant AAV1 vector, which includes the nucleic acid encoding the mutant of an AAV1 capsid protein.

The present invention is also directed to providing a pharmaceutical composition, which includes the recombinant AAV1 vector.

The present invention is also directed to providing a method of preventing or treating pulmonary hypertension, which includes administering a therapeutically effective amount of the pharmaceutical composition to a subject.

### [Advantageous Effects]

The present invention relates to a recombinant AAV1 capsid variant, which exhibits high gene delivery efficiency by specifically targeting pulmonary vessels. Particularly, the present invention relates to a recombinant viral vector including a nucleic acid encoding a mutant of an AAV1 capsid protein that is useful for the expression of a transgene in target pulmonary vessel cells when intratracheally delivered in an aerosol state, and thus can be used in prevention or treatment of a pulmonary vascular disease.

### [Description of Drawings]

FIG. 1 shows the 3D structure of a recombinant AAV1 vector (#2-3 variant).
FIG. 2A shows the cleavage map of the #2-3 variant.
FIG. 2B shows the cleavage map of a pHelper plasmid.
FIG. 2C shows the cleavage map of a pCMV GFP plasmid.
FIG. 2D shows the cleavage map of a pCMV LacZ plasmid.
FIG. 3 shows the entire base sequence of the recombinant AAV1 vector (#2-3 variant) represented by SEQ ID NO: 5 according to the present invention.

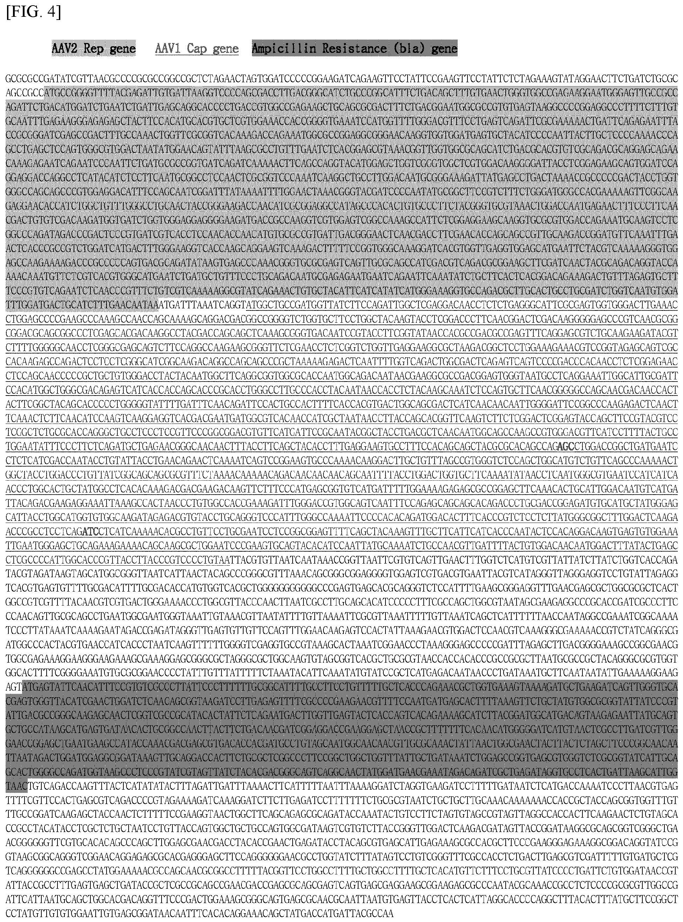

FIG. 4 shows the entire base sequence of a wild-type AAV1 vector.
FIG. 5 shows the comparison of genomic titers for the packaging efficiency of wild-type AAV1 and the recombinant AAV1 vector (#2-3 variant) of the present invention through quantitative PCR analysis.
FIG. 6 shows the result of analyzing the improvement in efficiency of transduction of human pulmonary artery smooth muscle cells (HPASMCs) by the proportion of GFP expressing cells among total cultured cells.
FIG. 7 shows the results of efficient local delivery in lung tissue in which LacZ expression has been confirmed by staining the lung extracted from an 8-week-old C57BL/6 male mouse with whole-mount β-galactosidase in order to confirm vascular specificity of the recombinant AAV1 vector (#2-3 vector) in lung tissue.
FIG. 8 shows the quantification of the pulmonary vascular smooth muscle cell targeting of the recombinant AAV1 vector (#2-3 variant).
FIG. 9 confirms the Brownian motion of the recombinant AAV1 vector (#2-3 variant) in 1xPBS (pH 7.4).
FIG. 10 confirms targeting and distribution to vascular smooth muscle cells after the intratracheal injection of the recombinant AAV1 vector (#2-3 variant).
FIG. 11 confirms LacZ expression of the recombinant AAV1 vector (#2-3 variant) in a blood vessel with a diameter of 100 µm or more through x-gal staining [V: Vessel, B: Bronchiole].

### [Modes of the Invention]

Hereinafter, the present invention will be described in further detail.

The present invention relates to a mutant of an adeno-associated virus serotype 1 (AAV1) capsid protein.

The term "adeno-associated virus" or "AAV" used in the present invention encompasses derivatives, viral subtypes, and naturally occurring and recombinant forms of all adeno-associated viruses used in gene therapy. Various serotypes of AAV may be used as recombinant gene delivery viruses for transducing a variety of different cell types. In addition to various serotypes of genomic sequences of AAV, the sequences of natural terminal repeats (TRs), Rep proteins, and capsid subunits are known in the art. These sequences can be found in the literature or in public databases such as GenBank. For example, GenBank Accession No. NC_002077(AAV-1), AF063497(AAV-1) may be referred to.

The term "serotype" used in the present invention refers to a subdivided part of AAV which can be identified by a serological or DNA sequencing method and distinguished by its antigenic properties.

The term "capsid" used in the present invention is a protein encoded by a cap gene present in the viral genome and constituting the outer shell of a virus. The wild-type AAV genome or cap gene encodes three types of capsid proteins (VP1, VP2, and VP3). In the specification, all of these are included as a capsid protein. The wild-type AAV1 capsid protein includes an amino acid sequence represented by SEQ ID NO: 1.

In one embodiment, the present invention relates to a mutant of an AAV1 capsid protein, and the mutant includes a mutant of an AAV1 capsid protein in which serine 430 is substituted with cysteine and isoleucine 647 is substituted with valine, compared to the amino acid sequence of the wild-type AAV1 capsid protein.

In one embodiment, the mutant of an AAV1 capsid protein according to the present invention includes or consists of an amino acid sequence (VP1) represented by SEQ ID NO: 3. VP2 corresponds to the amino acid sequence of SEQ ID NO: 3 from T138 to the end, and VP3 corresponds to the amino acid sequence of SEQ ID NO: 3 from M203 to the end.

In one embodiment of the present invention, the mutant of an AAV1 capsid protein according to the present invention is named #2-3.

The term "wild-type" used in the present invention refers to a type that can be most commonly found in wild populations. For mutant types, the wild type refers to the phenotype or individual considered to be basic. The wild-type is also called a "normal type." Meanwhile, the "mutant" used herein means a protein, virus, cell, or individual that is generated by the characteristic changes in mutated genes. In addition, the "mutant" used herein may also refer to a gene itself, which causes the mutation.

In one embodiment, the present invention includes a nucleic acid encoding the mutant of an AAV1 capsid protein. The nucleic acid of the present invention encodes the mutant of an AAV1 capsid protein. The nucleic acid of the present invention is prepared by substituting at least one base in the base sequence of the nucleic acid (cap gene) encoding the AAV1 capsid protein with another base. The nucleic acid of the present invention may be present in the form of DNA, or in cases, the form of RNA, or a chimera of DNA and RNA. In addition, the nucleic acid of the present invention also includes a complementary nucleic acid (e.g., cDNA). The nucleic acid of the present invention may be single-stranded, or double-stranded, and preferably, double-stranded.

In the present invention, a nucleic acid that encodes a mutant of anAAV1 capsid protein is not particularly limited, but in one embodiment, a nucleic acid having a base sequence represented by SEQ ID NO: 4 is exemplified.

The nucleic acid of the present invention may be operably linked with an appropriate control sequence. Control sequences include a promoter sequence, a polyadenylation sequence, a transcription termination sequence, an upstream regulatory domain, an internal ribosome entry site (IRES), and an enhancer. Promoter sequences include an inducible promoter sequence and a constitutive promoter sequence. Control sequences may be unique to the AAV from which the capsid protein is derived, and they may be foreign, natural, or synthetic sequences. The present invention also includes recombinant DNA that can express a mutant of an AAV1 capsid protein including the nucleic acid of the present invention.

The recombinant DNA is useful for delivering the nucleic acid of the present invention to cells in vitro, ex vivo, and in vivo, and imparting the ability of expressing a mutant of an AAV1 capsid protein to the corresponding cells. In addition, the cells to which the nucleic acid of the present invention is delivered are useful for producing recombinant AAV particles. The recombinant DNA may be used to deliver or introduce the nucleic acid of the present invention especially to eukaryotic cells, preferably, animal cells, and more preferably, mammal cells.

In the present invention, recombinant DNA may be prepared by harboring the nucleic acid of the present invention in DNA that is used as a vector. For example, as recombinant DNA, a plasmid, a phage, a transposon, a cosmid, episomal DNA, or a viral genome may be used.

For example, by harboring the nucleic acid (cap gene) encoding the mutant of the AAV1 capsid protein of the present invention in a plasmid, a packaging plasmid may be produced. The packaging plasmid may further include any nucleic acid sequence, such as a nucleic acid (rep gene) encoding a replicase (Rep) protein. Preferably, the rep gene includes AAV2-derived Rep.

In the nucleic acid sequence of the cap gene carried in a known packaging plasmid, even by substituting at least one base in a PLA2 domain-coding region with another base, recombinant DNA including the nucleic acid of the present invention may be prepared. There is no particular limitation on the packaging plasmid, but examples include a packaging plasmid carrying the cap gene, and preferably, a packaging plasmid carrying the cap gene and rep gene. In one example, in the present invention, a recombinant AAV1 vector, p #2-3, represented by SEQ ID NO: 5, which is a packaging plasmid carrying the nucleic acid (cap gene) encoding the mutant of the AAV1 capsid protein of the present invention and the rep gene, was constructed.

A method of introducing base substitution into the nucleic acid may be carried out by a known method, and may be accomplished, but not particularly limited to, by performing PCR using a commercially available reagent, e.g., a mutagenesis basal kit (TAKARA BIO INC.) according to the instructions contained in the kit.

Accordingly, the present invention includes a recombinant AAV1 vector, which includes a nucleic acid encoding the AAV1 capsid protein variant as a novel AAV1 #2-3 variant for gene therapy application.

The recombinant AAV vector of the present invention is useful for gene introduction into target cells. A gene introduced into the recombinant AAV vector of the present invention is strongly expressed in the target cells.

The "AAV vector" used herein refers to any vector that includes or is derived from a component of an adeno-associated virus (AAV) and is suitable for infecting any mammalian cell including human cells of many tissue types, such as the brain, heart, lung, skeletal muscle, liver, kidney, spleen or pancreas, regardless of in vitro or in vivo. The term "AAV vector" may be used to refer to an AAV-type viral particle (or virion) including at least a nucleic acid molecule encoding a protein of interest.

The "AAV virus," "AAV viral particle," or "rAAV vector particle" used herein refers to a viral particle consisting of at least one AAV capsid protein (e.g., all capsid proteins of wild-type AAV) and a polynucleotide rAAV vector encapsidated in a capsid. When a particle includes a heterologous polynucleotide (i.e., a polynucleotide other than a wild-type AAV genome, such as a transgene delivered to mammalian cells), it is typically referred to as an "rAAV vector particle" or simply an "rAAV vector." Accordingly, the generation of the rAAV particle must include rAAV generation, which is because such a vector is contained in the rAAV particle.

"Packaging" refers to a series of intracellular events causing the introduction of an AAV particle into an assembly and a capsid.

AAV "rep" and "cap" genes refer to polynucleotide sequences that replicate adeno-associated viruses (AAVs) and encode encapsidated proteins. AAV rep and cap are referred to as AAV "packaging genes" in the specification.

"Helper virus" for AAV refers to a virus that allows AAV (e.g., wild-type AAV) to be replicated and packaged by mammalian cells. A variety of helper viruses for AAV, for example, adenoviruses, herpes viruses, and poxviruses such as vaccinia, are known in the art. While subgroup C adenovirus serotype 5 was most frequently used, adenoviruses include several different subgroups. Numerous adenoviruses derived from humans, non-human mammals, and avians are known, and available from the depositories such as ATCC. Herpes viruses include, for example, not only herpes simplex virus (HSV) and Epstein-Barr virus (EBV), but also cytomegalovirus (CMV) and pseudorabies virus (PRV); and these are also available from the depositories such as ATCC.

"Helper virus function(s)" refer to function(s) encoded in the helper virus genome that allows AAV replication and packaging (along with other requirements for replication and packaging described herein). As described in the specification, "helper virus function" may be provided by various methods including providing a helper virus, or for example, providing a polynucleotide sequence encoding essential function(s) to producer cells in trans. For example, a plasmid or another expression vector, which includes a nucleotide sequence encoding one or more adenovirus proteins, is transfected into producer cells along with an rAAV vector.

In one embodiment, the AAV1 vector according to the present invention has an improved transduction profile for target tissue as compared to an AAV1 vector including a wild-type capsid protein. In other words, the AAV1 vector according to the present invention has obvious tissue targeting ability (e.g., tissue tropism).

The term "tropism" used herein means the specificity of an AAV capsid protein present in an AAV virus particle for infecting or transducing a specific type of cell or tissue.

The tropism of an AAV capsid for a specific type of cell or tissue may be determined by measuring the ability of AAV vector particles to transfect or transduce a specific type of cells or tissue due to the AAV1 capsid protein therein using a standard analysis method well known in the art, which is the same as described in examples of the present specification.

That is, "tropism" refers to the ability of an AAV vector or virion to infect one or more specific cell types. However, it can also include whether a vector serves to transduce one or more specific cell types. That is, tropism refers to the expression (e.g., transcription and in some cases, translation) of a sequence delivered by an AAV vector or virion, depending on the case and preferably, in cells, for example, the expression of heterologous nucleotide sequence(s) in the case of a recombinant virus, following the preferential introduction of an AAV vector or virion into specific cell or tissue type(s) and/or the preferential interaction with a cell surface facilitating entry into a specific cell or tissue type.

The term "transduction" used herein refers to the ability of an AAV vector or virion to infect one or more specific cell types. That is, transduction means that an AAV vector or virion is introduced into cells to deliver a genetic material contained in the AAV vector or virion to cells, thereby achieving expression from the vector genome. In some, but not all, cases, transduction and tropism may be correlated.

The AAV described in the present invention includes amino acid modification(s) in one or more capsid proteins, which imparts a new or enhanced tissue tropism property. An AAV1 modification according to the present invention targets a pulmonary vessel (e.g., a pulmonary artery).

The term "pulmonary tissue or pulmonary vascular tropism" used herein means tropism to lung tissue or pulmonary vessel.

In some embodiments, the pulmonary vascular tropism of a peptide-modified hybrid AAV capsid protein is further increased at least 5%, 10%, 20%, 30%, 40%, or 50% or more, compared to the pulmonary vascular tropism of a wild-type AAV capsid protein without a peptide.

In addition, the present invention may include a pharmaceutical composition including the recombinant AAV1 vector. The composition may further include a pharmaceutically acceptable carrier.

The "pharmaceutically acceptable carrier" includes any material that allows a component to retain biological activity without causing disruptive physiological reactions such as unintended immune reactions when combined with an active ingredient of the composition. Pharmaceutically acceptable carriers include water, phosphate buffered saline, emulsions such as an oil/water emulsion, and wetting agents. Compositions including these carriers are formulated by well known, conventional methods as used in Remington's Pharmaceutical Sciences, current Ed., Mack Publishing Co., Easton Pa. 18042, USA; A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., 7th ed., Lippincott, Williams, & Wilkins; and Handbook of Pharmaceutical Excipients (2000) A. H. Kibbe et al., 3rd ed. Amer. Pharmaceutical Assoc.

In one embodiment, the pharmaceutical composition according to the present invention may be a pharmaceutical composition for preventing or treating pulmonary arterial hypertension.

The term "treatment" used herein refers to the administration of an activating agent in any type of intervention or process performed on a subject or to a subject to reverse, alleviate, improve, inhibit, delay or prevent the progression, development, severity or relapse of a disease-related syndrome, complication, symptom, or biochemical sign. The treatment may be performed on a subject with a disease or a subject without a disease (e.g., for prevention).

In addition, in one embodiment, the present invention includes a method of preventing or treating pulmonary arterial hypertension, which includes administering a therapeutically effective amount of the pharmaceutical composition to a subject.

The "administration" used herein refers to the physical introduction of a therapeutic agent or a composition including a therapeutic agent to a subject using any one of various methods and delivery systems known by those of ordinary skill in the art. Preferable administration routes include parenteral administration routes such as intravenous, intraperitoneal, intramuscular, subcutaneous, intrathecal, intravitreal administration routes, for example, by injection or infusion. The phrase "parenteral administration" used herein generally refers to an administration method, other than intestinal and local administration by injection, which may be, but not limited to, intravenous, intraperitoneal, intramuscular, intraarterial, intrathecal, intralymphatic, intralesional, intracapsular, intraorbital, intracardiac, intradermal, transtracheal, subcutaneous, subepidermal, intravitreal, intraarticular, subcapsular, subarachnoidal, intraspinal, epidural, or intrasternal injection or infusion, or in vivo electroporation.

The term "therapeutically effective amount" used herein refers to an amount of a drug effective to "treat" a disease or disorder in a subject, or reduce the risk, potential, possibility or occurrence of a disease or disorder (e.g., pulmonary arterial hypertension) alone or in combination with a different therapeutic agent. The "therapeutically effective amount" includes the amount of a drug or therapeutic agent that provides partial improvement or benefit to a subject having a disease or disorder (e.g., the pulmonary arterial hypertension disclosed herein) or the risk of a disease or disorder. Accordingly, the "therapeutically effective amount" reduces the risk, potential, possibility or occurrence of a disease or disorder, or provides partial alleviation or reduction, and/or reduces at least one indicator (e.g., pulmonary arterial hypertension), and/or reduces at least one clinical symptom of a disease or disorder.

The term "subject" used herein includes a human or a non-human animal. The term "non-human animal" includes all vertebrates, including mammals, non-human primates, sheep, dog, cattle, chickens, and non-mammals such as amphibians and reptiles.

Hereinafter, the present invention will be described in further detail with reference to examples according to the present invention, but the scope of the present invention is not limited by the examples presented below.

### [Examples]

### Example 1: Selection of pulmonary vessel tropic AAV1 capsid protein variant using AAV library

### 1) Selection of AAV1 capsid protein variant

A plasmid pool was produced by inducing a random point mutation in the cap gene of each of wild-type AAV variants (AAV1, AAV2, AAV4, AAV6, AAV8, and AAV9) using error-prone PCR and inserting random 7mer/9mer into the 3-fold protrusion of each serotype. After forming a calcium-phosphate complex with 7 to 70 ng of the AAV plasmid library, 25 µg of pBluescript, and 25 µg of pHelper, the calcium-phosphate complex was transfected into AAV293 cells to perform AAV packaging, and an AAV library pool carrying the cap gene information of each variant was produced.

After respiratory anesthesia of an 8-week-old C57BL/6 male mouse with isofluorane, an approximately 1-cm incision was made in the skin of the tracheal area. 1×10¹¹ vg/100 µL of the AAV library pool in PBS was loaded in a PenWu micro-aerosolizer (BioJane, Shanghai, China) (length of intratracheal portion: 1.25", outer diameter: 700 µm, inner diameter: 430 µm), and intratracheal injection was performed by checking a needle passing through the airway.

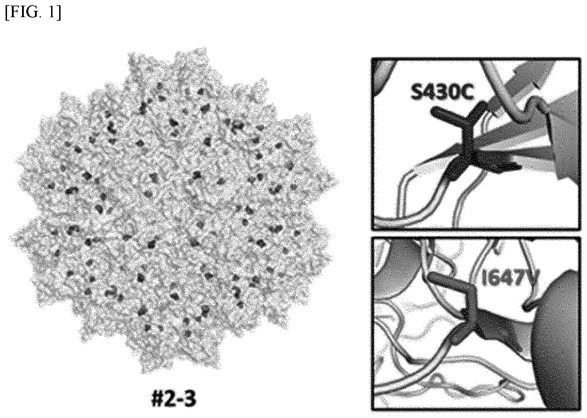

After one week, 0.9% saline was perfused through the heart, and the lungs were removed. After homogenization, DNA was extracted from the entire lungs using a DNA mini kit (Qiagen), the cap gene of AAV variants showing tropism to the entire lungs was amplified using the AAV cap gene-specific forward primer 5'-GCGGAAGCTTCGATCAACTACG-3' (SEQ ID NO: 7) and reverse primer 5'-CGCAGAGACCAAAGTTCAACTGA-3' (SEQ ID NO: 8). A pulmonary tropic AAV library pool was produced with the genes to perform intratracheal injection in the same manner as above (1×10¹¹ vg/100 µL). After one week, perfusion and lung extraction were performed, and after chopping the lung for 30 seconds into small pieces, cell dissociation was performed using collagenase II. Here, DNase I was added to prevent cell clustering caused by chromosomal DNA released from dead cells, resulting in minimized cell loss. After incubation at 37 °C for 4 to 6 hours, the total lung population in the form of single cells was obtained through pipetting, red blood cell lysis was performed while light was blocked at room temperature, and then the cells were transferred to FACS buffer through a cell strainer with a pore size of 70 µm. Afterward, to select AAV variants showing vascular tropism, 10 µg of an anti-alpha smooth muscle actin antibody (APC) was added, followed by incubation at 4 °C. Subsequently, APC positive cells were sorted to select blood vessel-related cells, and after cell lysis and DNA extraction, the cap gene of the AAV variants entering cells was amplified using primers ((5'-GCGGAAGCTTCGATCAACTACG-3': SEQ ID NO: 9) and (5'-CGCAGAGACCAAAGTTCAACTGA-3': SEQ ID NO: 10)). The amplified cap gene includes HindIII and NotI sequences at both ends, and subcloned into a pSub2 plasmid through HindIII/NotI restriction and ligation and electroporated into DH10β, and the plasmid was purified (Qiagen Plasmid Maxi Kit) and stored in the form of a blood vessel-tropic plasmid pool (pSub2 is a plasmid produced in the David Schaffer Lab (UC Berkeley) based on pSub201(ATCC) to subclone the cap gene using HindIII and NotI, Reference 1. Narendra Maheshri et al., Nature Biotechnology, 2006; 2. James T Koerber, Nature Protocols, 2006). Among various variants, as an AAV gene delivery system for preventing and treating pulmonary arterial hypertension selected through total three rounds of in vivo selection, the #2-3 variant was finally selected, confirming that the #2-3 variant has AAV1-based point mutations S430C and I647V (FIG. 1).

### 2) Production of recombinant AAV1 vector (AAV1 #2-3 variant)

### ① Production of packaging plasmid mutant

The production of multiple blood vessel-specific plasmid mutants for loading a reporter gene was completed by subcloning multiple blood vessel-tropic cap genes into a blood vessel-tropic plasmid pool using HindIII/NotI restriction enzymes in pXX2 (UC Berkeley, David Schaffer Lab) into which HindIII and NotI were introduced for cap gene insertion.

### ② Introduction of plasmid into AAV293 cells

### Production of AAV1 #2-3 variant

A calcium-phosphate complex formed of 17 µg of a mutant plasmid, 17 µg of a ITR flanked reporter gene (pCMV-GFP, pCMV-LacZ, or pCMV-FGF12-IRES-GFP), and 17 µg of pHelper was transfected into AAV293 cells, and after approximately 48 hours, only a cell pellet was collected and then intracellular AAV was extracted therefrom through freezing-thawing. Afterward, cell debris was removed through centrifugation, and 10 U/mL of benzonase was incubated at 37 °C for 30 minutes to remove a nucleic acid derived from virus-producing cells.

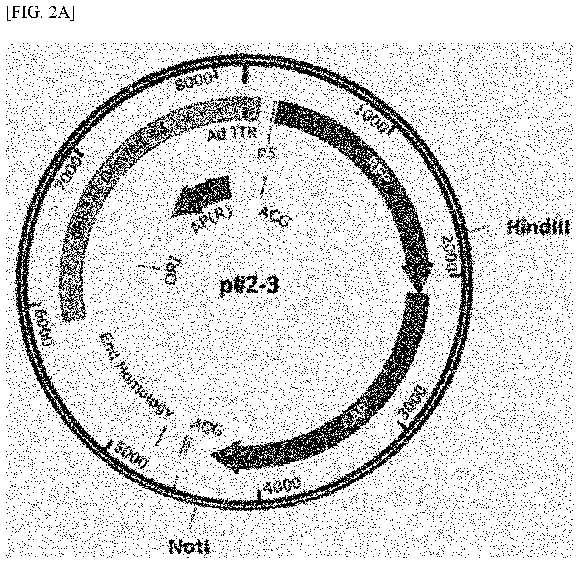

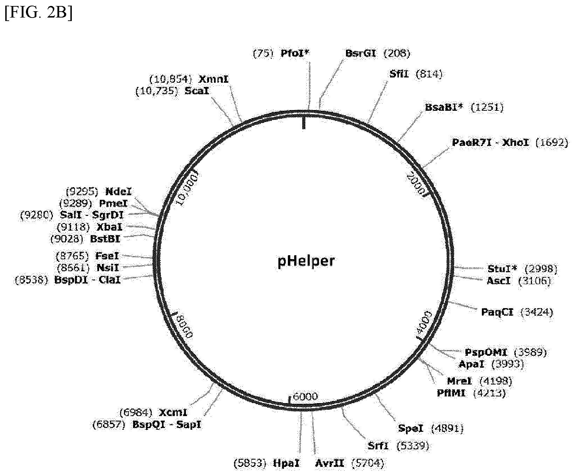

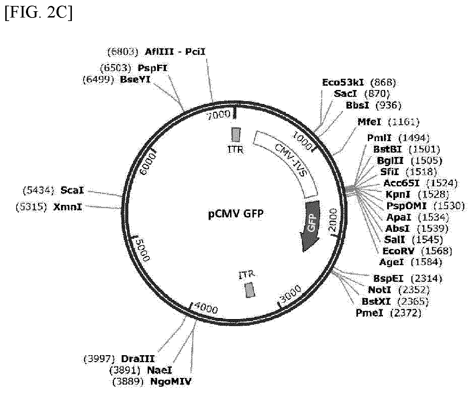

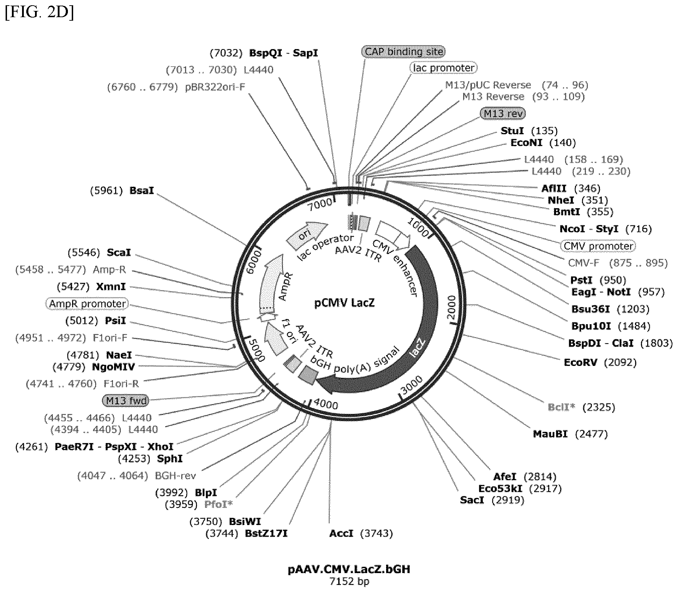

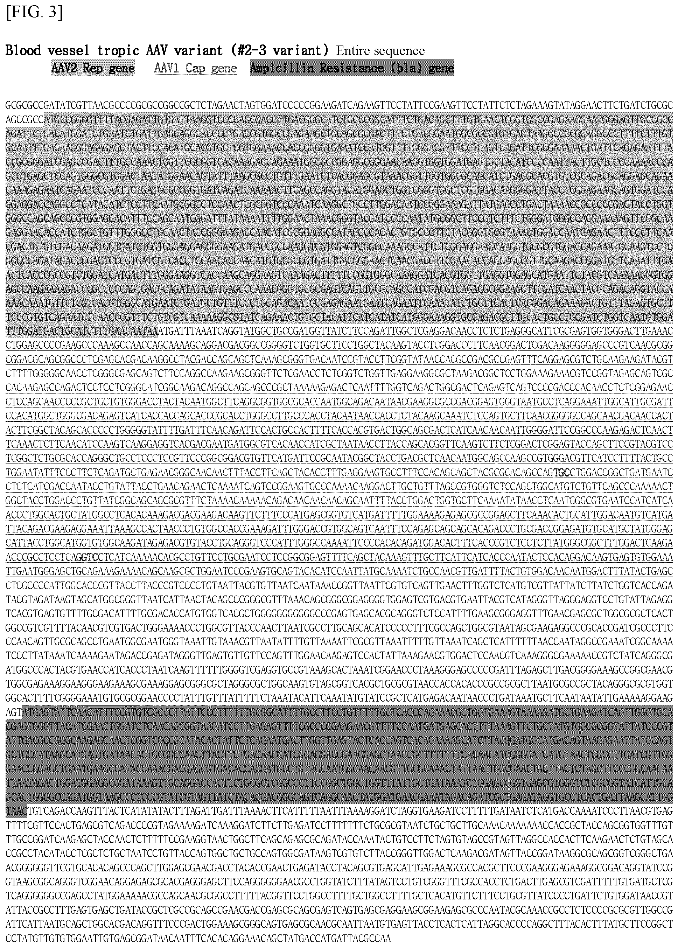

The cleavage map of the produced #2-3 variant is shown in FIG. 2, and the entire base sequence is shown in FIG. 3.

### Production of wild-type AAV1

A calcium-phosphate complex formed of 17 µg of a packaging plasmid containing the rep gene of AAV2 and the cap gene of AAV1, 17 µg of an ITR flanked reporter gene (pCMV-GFP, pCMV-LacZ, or pCMV-FGF12-IRES-GFP), and 17 µg of pHelper was transfected into AAV293 cells, and after approximately 48 hours, only a cell pellet was collected to extract intracellular AAV through freezing-thawing. Afterward, cell debris was removed through centrifugation, and 10 U/mL of benzonase was incubated at 37 °C for 30 minutes to remove a nucleic acid derived from virus-producing cells.

### 3) Purification of AAV1 #2-3 variant

An AAV solution was subjected to ultracentrifugation using an iodixanol gradient. 15%, 25%, 40%, and 54% iodixanol solutions were prepared and loaded sequentially in ultracentrifuge tubes, and the AAV solution was loaded on top of them. After tube sealing, ultracentrifugation was performed (42,000 RPM, 18 °C, 2 hrs) using an Optima XE-90 Ultracentrifuge (BECKMAN COULTER) and a Vti65.2 rotor. The AAV layer located between the 54% and 40% iodixanol gradients was extracted, and buffer exchange was performed with PBS buffer containing 0.01% Tween 20 using an Amicon^{®} Ultra-15 Centrifugal Filter (MWCO 100,000).

### 4) Measurement of AAV1 #2-3 variant titers

After extracting a viral genome by treating a virus having resistance to DNase I (5U) with protease K, the titers of wild-type AAV1 (wtAAV1) carrying CMV-FGF12-IRES-GFP and the AAV1 #2-3 variant were quantified by qPCR using quantitative PCR (qPCR) primers for CMV (5'-ATGGTGATGCGGTTTTGGCAG-3': SEQ ID NO: 11 and 5'-GGCGGAGTTGTTACGACATTTTGG-3': SEQ ID NO: 12) and the standards thereof.

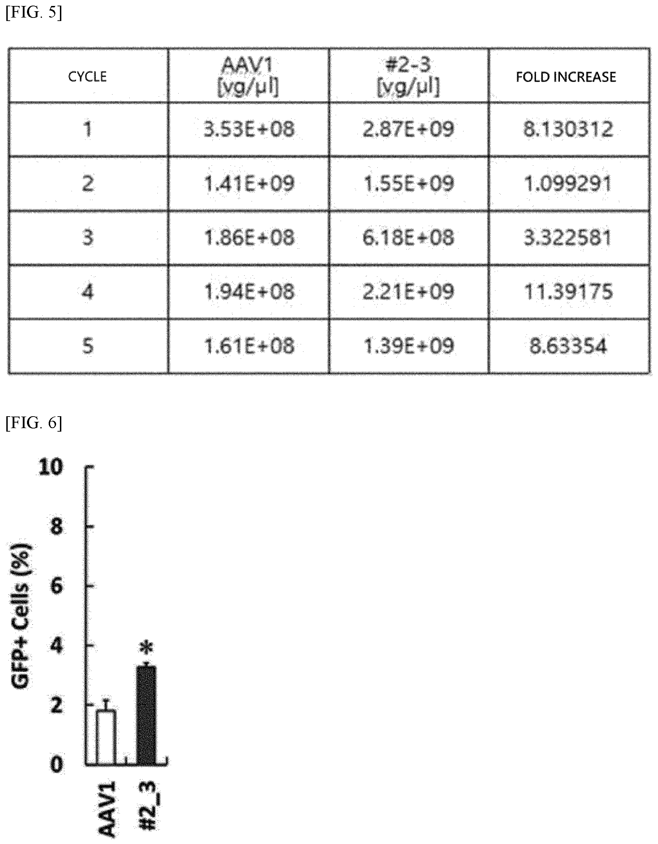

The packaging efficiency of the wild-type AAV1 and the recombinant AAV1 vector (#2-3 variant) was shown by comparing genomic titers, and the results are shown in FIG. 5. This shows that the packaging efficiency of the #2-3 variant is higher than that of the wild-type AAV1, indicating the potential to be maintained as an evolutionarily superior organism.

### Example 2: Confirmation of recombinant AAV1 variant infection

### 1) In vitro experiment

For analysis of gene delivery efficiency and location as a reporter gene, the wild-type AAV1 and the #2-3 variant, which carried CMV-GFP, were packaged, and used to infect human pulmonary arterial smooth muscle cells (HPASMCs; 2×10⁴ cells/20 µL).

CMV-FGF12-IRES-GFP was carried in each virus, and when GFP was carried, after 48 hours of HPASMC infection (MOI 10,000), the proportion (%) of GFP-expressing cells among total cultured cells was analyzed through flow cytometry to confirm infection ability. The results are shown in FIG. 6.

FIG. 6 shows the improvement in HPASMC transduction efficiency by the proportion of GFP-expressing cells among total cultured cells, showing that the #2-3 variant has excellent gene delivery efficiency to vascular cells and excellent tropism to human primary cells (HPASMCs) compared to the wild-type AAV1.

### 2) In vivo experiment

Each of the AAV1 wild-type and the #2-3 variant, which carries CMV-LacZ, was packaged and injected intratracheally into an 8-week-old C57BL/6 male mouse in the form of an aerosol (5×10¹¹ vg/100 µL).

To confirm the lung tissue specificity of the recombinant AAV1 vector (#2-3 variant), the result of confirming LacZ expression by staining the lung extracted from the 8-week-old C57BL/6 male mouse one week after the injection with whole-mount β-galactosidase is shown in FIG. 3, and this confirmed that the recombinant AAV1 vector (#2-3 variant) was effectively delivered locally to the lung tissue.

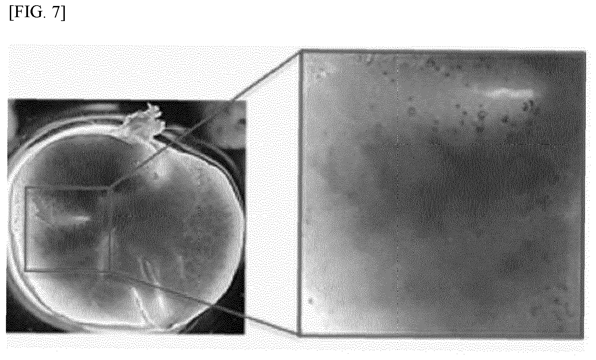

In addition, to confirm the vascular specificity of the recombinant AAV1 vector (#2-3 variant) in lung tissue, LacZ expression in the pulmonary vessels was checked through x-gal staining by extracting the lung from the 8-week-old C57BL/6 male mouse one week after injection. The results are shown in FIG. 7. This revealed that the recombinant AAV1 vector (#2-3 variant) was effectively delivered to the pulmonary blood vessels.

### Example 3: Confirmation of pulmonary vascular smooth muscle cell targeting efficiency of recombinant AAV1 variant

To evaluate vascular smooth muscle cell targeting efficiency in lung tissue, viral genomes entering several cells were intended to be quantified at a single cell level. The LacZ-carrying #2-3 variant and wtAAV1 were packaged and intratracheally injected (5×10¹¹ vg/100 µL) into 8-week-old C57BL/6 mice. Three or seven days after injection, the lungs were extracted and subjected to single cell dissociation, and then α-sma-conjugated antibodies (α-sma-Alexa488, abcam, ab184675) for each cell sorting were incubated at 4 °C overnight.

The ratio of viral genomes entering each cell compared to the initial injection dose was calculated, the fold change of the #2-3 variant compared to wtAAV1 was confirmed by normalization to the wtAAV1 value (*p-value<0.01).

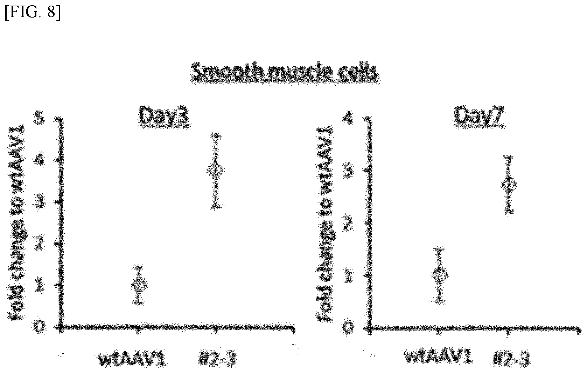

As shown in FIG. 8, there was a tendency that the ratios of viral genomes in the samples that had experienced smooth muscle cell sorting using the α-sma-conjugated antibodies increased 3.7±0.9 fold on Day 3 and 2.7±0.5 fold on Day 7 in the #2-3 variant compared to wtAAV1.

### Example 4: Confirmation of mobility of recombinant AAV1 variant

After the intratracheal injection of the #2-3 variant and wtAAV1 in the form of aerosol, they reach vascular smooth muscle cells through various barriers such as the tracheal wall, the extracellular matrix, and the vessel wall. One of the reasons that made this possible was the tropism of the #2-3 variant, and it was proven that the excellent mobility of AAV1 was caused by Brownian motion in a liquid phase (PBS phase).

### 1) 1xPBS, static state

As a result of observing a trajectory in a static state by dispersing wtAAV1 or the #2-3 variant at a concentration of 1.0E+09 vg/mL under a 1xPBS (pH 7.4) condition, the #2-3 variant showed diffusion-type movement compared to wtAAV1.

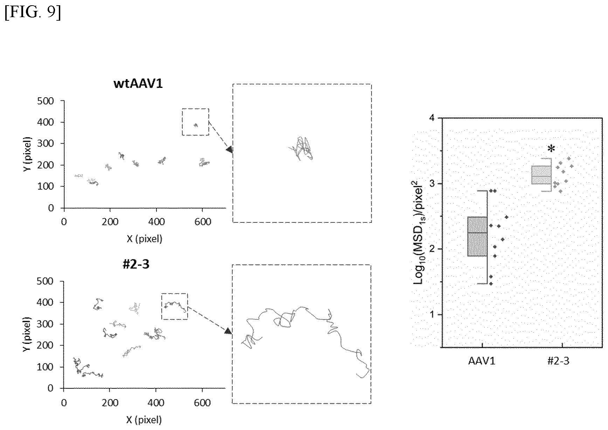

As a result of measuring the mean squared displacement (MSD) using the given equation [msd(r) = <Δr(τ)²> = <[r(t+τ) - r(t)]²>, wherein r(t) indicates a vector location at time t, τ indicates the lag time between two locations (the difference in trajectories obtained for each vector)] and summarizing the values by log₁₀(MSD₁ₛ)/pixel², it was found that the value of the #2-3 variant (3.12±0.17) showed a statistically significant increase compared to the value of the AAV1 (2.21±0.49) (*p-value<0.01). That is, this showed that the distance moved by the #2-3 variant under 1xPBS and pH 7.4 conditions was increased compared to that of wtAAV1 (FIG. 9).

Brownian motion refers to the irregular movement of particles in a liquid or gas, and diffusion refers to the movement of particles moving in a relatively directional manner. Diffusion can be considered a macroscopic expression of Brownian motion, so diffusion patterns can be predicted by calculating the average movement of Brownian motion.

That is, compared to AAV1 showing a trajectory that moves irregularly and stays in place, the trajectory of the #2-3 variant showed relatively linear movement, which is diffusion-type movement.

### Example 5: Confirmation of pulmonary vascular tropism of recombinant AAV1 variant

### 1) Confirmation of distribution of recombinant AAV1 variant in lung tissue

An 8-week-old C57BL/6 male mouse was subjected to intratracheal injection of Alexa594-tagged wtAAV1 or #2-3 variant (3×10¹¹ vg/100 µL), and 48 hours after injection, the lungs were extracted after anesthesia and perfusion through the heart. After 4% PFA fixation and sectioning, the colocalization of AAV distribution in the arteriole was confirmed using α-sma-antibodies (Alexa488).

As a result of observing the AAV distribution 24 hours after intratracheal injection, it was observed that an AAV vector tagged with Alexa594 (a fluorescent material conjugated to the AAV capsid) moved from the bronchiole to the arteriole in the #2-3 variant.

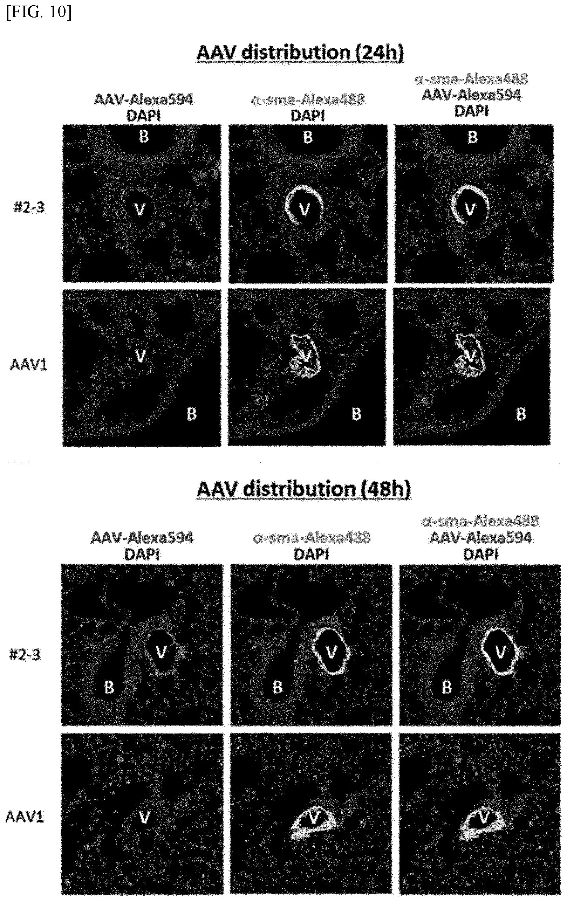

At the 48h time point after intratracheal injection, it was confirmed that most of the #2-3 vectors moved from the bronchiole to the arteriole and the AAV vectors are localized on the vessel wall, and also confirmed that the AAV vectors are colocalized with the α-sma antibodies, which are a smooth muscle cell marker in blood vessels, thereby confirming the pulmonary vascular tropism of the #2-3 vector. In contrast, it was confirmed, in the case of wtAAV1, at the 48h time point, a large quantity of AAV vectors are present in an air sac area of the perivascular space, but the colocalization with the α-sma antibodies was not shown (FIG. 10).

That is, as a result of confirming the movement of fluorescence-tagged viruses in lung tissue, wtAAV1 remained around the trachea or did not reach the blood vessel, whereas the #2-3 variant showed very smooth and fast movement toward the blood vessel through the trachea and the gene of the #2-3 vector was intensively found in vascular cells.

### 2) Confirmation of pulmonary vascular tropism by LacZ expression

An 8-week-old C57BL/6 male mouse was subjected to the intratracheal injection (3×10¹¹ vg/100 µL) of LacZ-carrying wtAAV1 or #2-3 variant, and after two weeks, X-gal staining was performed after anesthesia, perfusion through the heart, and sectioning. Compared to wtAAV1, the #2-3 variant showed LacZ expression in the vascular area.

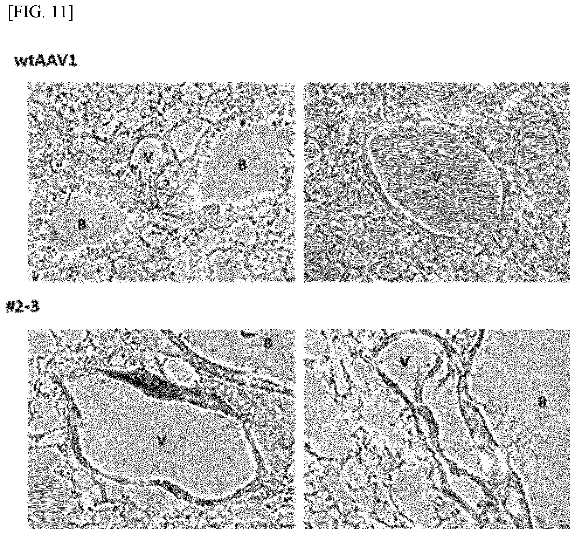

As shown in FIG. 11, the #2-3 vector had better mobility than wtAAV1, so it showed an improvement in the rate of physically reaching the vascular area in the lung tissue through the trachea and the extracellular matrix and showed vascular tropism, which is localization around blood vessels. Therefore, the recombinant AAV1, #2-3 vector, is expected to be used as an AAV vector for treating and preventing a pulmonary vessel-targeting disease.

## Claims

1. A mutant of an adeno-associated virus serotype 1 (AAV1) capsid protein, wherein serine 430 is substituted with cysteine and isoleucine 647 is substituted with valine compared to the amino acid sequence of a wild-type AAV1 capsid protein.

2. The mutant of claim 1, which has the amino acid sequence represented by SEQ ID NO: 3.

3. A nucleic acid encoding the mutant of an AAV1 capsid protein of claim 1.

4. The nucleic acid of claim 3, which has the base sequence represented by SEQ ID NO: 4.

5. A recombinant AAV1 vector comprising the nucleic acid encoding the mutant of the AAV1 capsid protein of claim 3.

6. The vector of claim 5, wherein the AAV1 vector has an improved transduction profile for target tissue as compared to a wild-type AAV1 vector.

7. The vector of claim 6, wherein the target tissue is a pulmonary vessel.

8. The vector of claim 7, wherein the pulmonary vessel is a pulmonary artery.

9. The vector of claim 5, which is represented by SEQ ID NO: 7.

10. A pharmaceutical composition comprising the recombinant AAV1 vector of claim 5.

11. The pharmaceutical composition of claim 10, further comprising a pharmaceutically acceptable carrier.

12. The pharmaceutical composition of claim 10, which is for preventing or treating pulmonary arterial hypertension.

13. The pharmaceutical composition of claim 10, which is administered by intravenous, intraperitoneal, intramuscular, intraarterial, intrathecal, intralymphatic, intralesional, intracapsular, intraorbital, intracardiac, intradermal, transtracheal, subcutaneous, subepidermal, intravitreal, intraarticular, subcapsular, subarachnoidal, intraspinal, epidural, or intrasternal injection.

14. A method of preventing or treating pulmonary arterial hypertension, comprising:
administering a pharmaceutical composition comprising a therapeutically effective amount of the recombinant AAV1 vector of claim 5 to a subject.

15. The method of claim 14, wherein the composition is administered by intravenous, intraperitoneal, intramuscular, intraarterial, intrathecal, intralymphatic, intralesional, intracapsular, intraorbital, intracardiac, intradermal, transtracheal, subcutaneous, subepidermal, intravitreal, intraarticular, subcapsular, subarachnoidal, intraspinal, epidural, or intrasternal injection.
